# EUROPÄISCHE PATENTANMELDUNG

(11) **EP 4 233 886 A1**
(43) Veröffentlichungstag der Anmeldung: **30.08.2023**
(21) Anmeldenummer: 22216672.0
(22) Anmeldetag: 24.12.2022
(51) Int. Cl.: A61K 36/185, A61P 35/00

(54) **PHARMAZEUTISCHE ZUSAMMENSETZUNG ZUR BEHANDLUNG EINES HIRNTUMORS**

(71) Anmelder: Patentpool Target GmbH, 80331 München (DE)
(72) Erfinder: Burda, Renate, 82054 Sauerlach (DE); M rth-Kretschmer, Jenny, 21244 Buchholz in der Nordheide (DE); Sand, Elisabeth, 91567 Rauenzell/Herrieden (DE); Weickmann, Dirk, 91567 Rauenzell/Herrieden (DE)
(74) Vertreter: Reich, Jochen

(57) **Zusammenfassung**

Die Erfindung betrifft eine pharmazeutische Zusammensetzung zur Behandlung eines Hirntumors, enthaltend in einer pharmazeutisch wirksamen Menge Peptidtoxin aus Pflanzen der Gattungen Dendrocnide und/ oder Urera und/ oder Nanocnide und/ oder Girardinia; und Gesamtgift oder Teile des Giftes von Pflanzen der Gattung Laportea sowie die Verwendung der pharmazeutischen Zusammensetzung zur Behandlung eines Hirntumors, ein Verfahren zur Herstellung der pharmazeutischen Zusammensetzung sowie ein Kit of parts enthaltend die pharmazeutische Zusammensetzung.

## Beschreibung

Die Erfindung betrifft eine pharmazeutische Zusammensetzung zur Behandlung eines Hirntumors, enthaltend in einer pharmazeutisch wirksamen Menge Peptidtoxin aus Pflanzen der Gattungen Dendrocnide und/ oder Urera und/ oder Nanocnide und/ oder Girardinia; und Gesamtgift oder Teile des Giftes von Pflanzen der Gattung Laportea sowie die Verwendung der pharmazeutischen Zusammensetzung zur Behandlung eines Hirntumors, ein Verfahren zur Herstellung der pharmazeutischen Zusammensetzung sowie ein Kit of parts enthaltend die pharmazeutische Zusammensetzung.
- Die vorliegende Erfindung betrifft eine pharmazeutische Zusammensetzung, die Verwendung der pharmazeutischen Zusammensetzung zur Behandlung eines Hirntumors, ein Verfahren zur Herstellung der pharmazeutischen Zusammensetzung sowie ein Kit of parts, enthaltend die pharmazeutische Zusammensetzung.
- In Deutschland treten pro Jahr etwa 9000 Fälle bösartiger Hirntumore auf. Dabei handelt es sich um Astrozytome mit Malignitätsgrad II und III, sowie Glioblastome des Grades IV und Glioblastoma multiforme. Ein Hirntumor wird auch heute noch meist als Nebenbefund diagnostiziert. Regelmäßig treten aber auch Blutungen im Schädel- und Hirnbereich der Patienten auf, die mit Bewusstlosigkeit und/ oder starken, teilweise sehr starken Schmerzen einhergehen. Ein Klinikaufenthalt wird dadurch unumgänglich.
- Die Behandlung von Hirntumoren gestaltet sich als äußerst schwierig. Insbesondere bei Auftreten von Glioblastomen sind die Prognosen für den Patienten meist nicht positiv. Durch eine operative Tumorentfernung kann die Überlebenszeit um einige Monate verlängert werden und die Symptome gelindert werden. Eine dauerhafte Heilung ist jedoch höchst selten und bei Glioblastomen aus schulmedizinischer Sicht nahezu unmöglich.

- Derzeit werden Tumore, als die gefährlichsten und gefürchtetsten Krankheiten unserer Zeit angesehen. Sie werden auf eine sehr radikale und den Patienten wenig schonende Weise bekämpft. Als einfache kennzeichnende Schlagworte können hier gelten:
   Stahl, Strahl und Chemotherapie.
- Das bedeutet einmal, dass Tumore, falls einigermaßen erreichbar, im Prinzip operativ mit dem Skalpell herausgeschnitten, durch eine breitgefächerte Bestrahlung zerstört, oder über eine sogenannte Chemotherapie mit, auch gesunde Zellen angreifenden, aggressiven Chemotherapeutika/Zytostatika zerstört werden. Sowohl bei normalen Behandlungen mit dem Skalpell als auch mit ionisierender Strahlung ist eine räumliche Begrenzung des Operationsgebiets nicht möglich. Es werden zwangsläufig auch gesunde Körperzellen vernichtet. Die unerwünschten Nebenwirkungen der Chemotherapie sind allgemein bekannt, ebenso wie die Nachteile einer Radiotherapie.
- Impfstoffe stehen nicht zur Verfügung und sind aus zellbiologischer und biochemischer Sicht eher kontraproduktiv was den Einsatz bei Erkrankungen mit Zellentartungen betrifft.
- Insbesondere die operative Entfernung von Hirntumoren hat jedoch weitere Nebenwirkungen. Häufig wird eine zweite Operation des Patienten notwendig. Bei der ersten Operation und Entfernung des Hirntumors wird eine große Narbenfläche erzeugt. Dort können sich neben gesunden Gliazellen auch kranke, d. h. maligne Gliazellen anlagern. Dies sind häufig Zellen, die aus dem Tumor stammen und Metastasen erzeugen. Dadurch ist ein diffuses Rezidiv vorprogrammiert. Dieses kann nicht mehr auf herkömmlichen Behandlungswegen behandelt werden. Daher ist dieses für einen Patienten oft letal.
- Nach einer Operation erfolgt üblicherweise eine Strahlentherapie und/ oder eine Chemotherapie. Bei der Chemotherapie wird häufig Cisplatin in verschiedenen Kombinationen verwendet. Seit Anfang 2002 wird auch das oral einzunehmende Temozolomid verwendet. Auch IMATINIB ist ein oral einzunehmendes Zytostatikum, das Anwendung findet. Neben den bekannten Nebenwirkungen wie Erbrechen, Unwohlsein, Depressionen, durch die Chemotherapie hervorgerufenen Krebs und Nervenleiden besteht bei den oben genannten Mitteln das Problem, dass sie zur Behandlung des Hirntumors die Blut-Hirn-Schranke überwinden müssen. Die Wirkstoffe durchschreiten die Blut-Hirn-Schranke üblicherweise lediglich mit 0,4 bis 5%. Die Zusammensetzung und Funktionsweise der Blut-Hirn-Schranke sind bis heute nur in Ansätzen verstanden. Diese Barriere spielt beim Schutz des Gehirns vor schädlichen Stoffen eine Rolle, ermöglicht andererseits aber auch eine geregelte Energiezufuhr.
- Unser Körper besteht aus einzelnen Organsystemen und Organen, die für ihre Funktion unterschiedliche, aber konstante Bedingungen zum Beispiel an Nährstoffen, Hormonen oder Elektrolyten benötigen. Alle Organe sind durch den Blutkreislauf miteinander verbunden. Da im Blut alle Bestandteile für die Versorgung, aber auch für die Entschlackung des Körpers enthalten sind, müssen Filtersysteme dafür sorgen, dass für die einzelnen Organsysteme nur die benötigten Stoffe durchgelassen beziehungsweise teilweise zurückgehalten werden. In diesem Zusammenhang kennt man unter anderem die Blut-Gewebe-Schranke, auch als Blut-Parenchym-Schranke bezeichnet, die Blut-Leber-Schranke, die Blut-Liquor-Schranke, die Blut-Hirn-Schranke, die Liquor-Hirn-Schranke, die Blut-Nerven-Schranke, die Blut-Retina-Schranke und die Plazenta-Schranke.
- Bei diesen Filtermechanismen wird durch einen so genannten Schrankeneffekt der Übertritt bestimmter Stoffe aus der Blutbahn in das jeweilige Organsystem verhindert oder eingeschränkt, wenn diese Organsysteme die Bestandteile nicht oder nur in geringerer Konzentration benötigen.
- Diese "Schranken" sind keine selbständigen Organe, sondern sie werden aus einer Vielzahl von Zellen und Zellzwischenräumen gebildet, die die Blutgase, die Nährstoffe und bestimmte Chemikalien durchlassen oder als Endothelporen Makromoleküle zurückhalten. Sie können auch als Lipidmembranen in der Gefäßwand hemmend auf den Durchtritt nicht lipidlöslicher Stoffe wirken oder eine selektive Wirkung aktiver Transportprozesse in den Kapillaren bewirken. Das Gehirn und das Nervengewebe werden durch zwei Filtersysteme geschützt, die Blut-Liquor-Schranke und die Blut-Hirn-Schranke.
- Das Vorhandensein und die Funktion der so genannten Blut-Hirn-Schranke ist schon seit über 100 Jahren bekannt und von Paul Ehrlich bereits 1885 im Experiment nachgewiesen worden. Innerhalb des Zentralnervensystems sind die Räume zwischen den Neuronen fast völlig durch Gliazellen und ihre Ausläufer ausgefüllt. Der gesamte Stoffwechsel der Nervenzellen geht über diese Glia- oder Endothelzellen. Sie dienen zum Einbau der Nervenzellen und Nervenfasern und zu ihrer Ernährung und Isolation. Eine Form der Gliazellen sind die Astrocyten. Sie besitzen zahlreiche Fortsätze, mit denen sie sich an der Wand der Kapillaren befestigen und eine die Kapillaren allseitig umgebende, nahezu spaltenlose Endothelauskleidung bilden. Diese Endothelzellen sind durch Verbindungselemente, die "tight junctions", verknüpft und mit einer selektiven Stoffdurchlässigkeit ausgestattet, die nur Partikel mit einem Durchmesser kleiner 20 nm passieren lässt. Auf diese Weise geht der gesamte Stoffwechsel der Nervenzellen über dieses endotheliale Geflecht, das die im Blut vorhandenen Substanzen gleich einem biologischen Filter bei Bedarf durchlässt, aber für die Gehirnfunktion schädliche Substanzen vom Nervensystem fernhält.
- Dieses Endothelgeflecht und die Endothelzellen, die die Kapillaren als eine Basalmembran auskleiden, werden als Blut-Hirn-Schranke bezeichnet. Ungehindert durchgelassen werden Sauerstoff, Kohlendioxid, D-Glukose, D-Hexose, einige L-Aminosäuren und lipidlösliche Stoffe, die für die Versorgung des Gehirns notwendig sind. Ebenso werden Abbauprodukte ins Blut abgegeben. Eine gewisse Barriere stellen die Endfortsätze der Astrocyten für zahlreiche Stoffe wie bestimmte Hormone, nicht lipidlösliche, wasserlösliche und chemische Substanzen sowie Proteine dar und sichern dadurch die Aufrechterhaltung eines konstanten Milieus für die Neuronen des Nervensystems.
- Das Zellgefüge der Astrozcyten ist so angeordnet, dass es eine effektive Abschottung gegen höhermolekulare Substanzen und Organismen bildet. Es ist aber auch unter Normalbedingungen nicht völlig dicht, sodass einige Partikel immer diese Schranke durchdringen können. Bei Infektionen, Traumen, Entzündungen, Vergiftungen, Hypoxidosen, Fieber und im Bereich von Tumoren werden die engen Verbindungen, die tight junctions, zwischen den Endothelzellen durch die Schwellung der Astrocythen aufgedehnt und deutlich durchlässiger für andere Stoffe. Die Änderung der Lichtungsweite erfolgt durch Quellung und Entquellung der Endothelzellen. Auch die Basalmembran der Kapillaren ist keine geschlossene Schicht. Je nach der Dichte des Fasernetzes entstehen Poren in der Membran, die aktiv am Stoffaustausch beteiligt sind.
- Schon lange vor der Möglichkeit einer Antibiotikabehandlung hat man die Durchlässigkeit der Blut-Hirn-Schranke durch künstliche Fiebererzeugung ähnlich dem Vorgang bei Infektionen gesteigert und zur Therapie der Syphilis des Zentralnervensystems und zur Schockbehandlung in der Psychiatrie ausgenutzt, indem man Medikamente damit direkt an das Gehirn herangeführt hat. Nach dem Ende der Einwirkung der die Blut-Hirn-Schranke beeinflussenden Bedingungen bildet sich die zeitweise Durchlässigkeit wieder zurück.
- Für die Chemotherapie von Patienten mit Hirnmetastasen solider Tumoren wird derzeit Termozolomid ein lipophiles Alkylans, klinisch erforscht. Es überwindet die Blut-Hirn-Schranke und erhöht die Strahlensensibilität von Tumoren bei simultaner Radiotherapie.
- Im Gegensatz hierzu wurde aber auch versucht, eine Krebstherapie auf subtilere Weise auf Basis von Naturstoffen zu ermöglichen.
- Es werden hierzu, unter anderem, viele aus giftigen Lebewesen isolierte, stark wirksame Stoffe in therapeutischen Dosen als Arzneistoffe genutzt.
- Die Nutzung dieser biogenen Gifte begann schon früh in der Geschichte der Menschheit. Zur gefahrlosen Anwendung dieser Gifte waren jedoch von Anfang an gewisse Grundkenntnisse über deren Behandlung und Wirksamkeit erforderlich. Die weiter durchgeführten Versuche, die Zusammensetzung des chemischen Aufbaus biogener Gifte zu entschlüsseln, führten später zur gezielten Suche bestimmter Wirkstoffe als eigentliche Verursacher beobachteter Wirkungen.
- Einen gewaltigen Aufschwung in der Trenntechnik, dem Weg zur Ermittlung von Wirkstoffen zur Bekämpfung von Krankheiten, machte die Entwicklung chromatographischer Verfahren in der Mitte des 20. Jahrhunderts möglich. Ausgehend von der Verteilung zwischen einer mobilen und einer stationären flüssigen Phase, von der Adsorption, den Molekülsiebeffekten, dem Ionenaustausch, der Affinität (insbesondere von Proteinen) zu bestimmten chemischen Verbindungen (z.B. Enzymsubstraten) und der Beweglichkeit geladener Moleküle im elektrischen Feld, wurde eine Vielzahl neuer Trenntechniken entwickelt.
- Gerade in neuerer Zeit wurden aus biogenen Giften (aus Pilzen, Bakterien, Pflanzen und Tieren), viele pharmazeutische Wirkstoffe isoliert und weiterentwickelt.
- So ist aus der PCT/EP00/12902 ein pharmazeutischer Wirkstoff bekannt, bei dem gefunden wurde, dass Bestandteile der Spinnengifte von Spinnen der Familie Sicariidae zur Behandlung von Tumorerkrankungen verwendet werden können. Es werden hierbei in der Hauptsache ein Peptidtoxin aus dem Gift dieser Spinnenart, eine weitere, aus dem Gift gewonnene, antagonistisch wirkende Substanz und/ oder eine Kombination dieser Bestandteile medizinisch genutzt. Vorgeschlagen wird die Verwendung von Peptidtoxinen aus beispielsweise der Gattung Sicarius zur Behandlung von Mammakarzinomen, Lungenkarzinomen, Adenokarzinomen, Leberkarzinomen sowie Melanomen. Die Behandelbarkeit von Hirntumoren wie Astrozytomen und Glioblastomen wird dagegen nicht erwähnt. Konsequenterweise wird auch das Problem der Überwindung der Blut-Hirn-Schranke nicht diskutiert.
- Die in der PCT/EP00/12902 beschriebenen Wirkstoffe können zur Behandlung von Tumorerkrankungen sowie parallel bzw. unterstützend bei Tumoroperationen eingesetzt und Rest-Tumorgewebe zerstört werden. Bei der Therapie können genetisch veränderte Körperzellen (Tumorzellen) zerstört werden, da der betreffende Wirkstoff die veränderte Oberflächenstruktur solcher Zellen erkennt und komplikationsfrei abtötet. Das Gesamtgift dieser Spinnenarten, sozusagen ein Cocktail verschiedener Substanzen, ist auf Grund seiner bereits in geringen Dosen letalen Wirkung nicht pharmazeutisch einsetzbar.
- Dieser bekannte Wirkstoff wirkt jedoch in vivo in keiner Kombination bei einem Hirntumor, insbesondere nicht bei einer besonderen Art von Hirntumor, nämlich einem Oligodendrogliom oder Oligodendrozytom. Zudem ist es für eine erfolgreiche Therapie notwendig, dass dieser Wirkstoff zu großen Teilen die Blut-Hirn-Schranke überwindet.
- Eine weitere Kombination von Giftstoffen ist in der PCT/EP2006/063281 offenbart. Die darin offenbarte Kombination verschiedener Giftstoffe neutralisiert sich jedoch teilweise gegenseitig, so dass sich die erwünschte tumorzellzerstörende Wirkung nur unzureichend einstellt.
- Es hat sich nämlich überraschenderweise herausgestellt, dass die Peptidtoxine von Loxosceles die Peptidtoxine von Latrodectus abbauen, so dass es zu keiner signifikanten Durchdringung der Blut-Hirn-Schranke der Loxosceles-Peptidtoxine kommt. Auf einem Elektrophoresegel zeigten sich nach einer Mischung der Peptidtoxine von Latrodectus und Loxosceles keine distinkten Banden, sondern lediglich ein Schmier, verursacht durch die Bruchstücke des Peptidverdaus der Latrodectus-Peptidtoxine. Spinnen der Gattung Loxosceles gehören wie Spinnen der Gattung Sicarius zur Familie der Sicariidae. Aufgrund der engen Verwandtschaft beider Spinnengattungen war deshalb auch für eine Mischung aus Peptidtoxinen der Gattung Sicarius mit solchen der Gattung Latrodectus keine Wirkung zu erwarten.

- Es ist deshalb eine Aufgabe der vorliegenden Erfindung, eine Zusammensetzung bereitzustellen, die unter möglichst effektiver Überwindung der Blut-Hirn-Schranke eine komplikationslose Abtötung von kanzerösen Körperzellen aus dem Bereich des Hirngewebes, speziell des seltenen Hirntumors Oligodendrogliom, bewirkt.
- Eine andere Aufgabe der vorliegenden Erfindung ist es, ein Verfahren bereitzustellen, das die Herstellung einer pharmazeutischen Zusammensetzung zur Behandlung von Hirntumoren ermöglicht.
- Es ist eine weitere Aufgabe der vorliegenden Erfindung, eine Kombination von Wirkstoffen bereitzustellen, die eine wirksame Behandlung von Hirntumoren ermöglicht.
- Diese Aufgaben werden durch die Merkmale der unabhängigen Ansprüche gelöst. Bevorzugte Weiterbildungen und Ausgestaltungen der Erfindung finden sich in den Unteransprüchen.
- Ein erster Aspekt der vorliegenden Erfindung betrifft daher eine pharmazeutische Zusammensetzung zur Behandlung eines Hirntumors, enthaltend in einer pharmazeutisch wirksamen Menge

### 1. a) Peptidtoxin aus Pflanzen der Gattung Dendrocnide und/ oder Pflanzen der Gattung Urera und/ oder Pflanzen der Gattung Nanocnide und/ oder Pflanzen der Gattung Girardinia und

### 2. b) Peptidtoxin aus Pflanzen der Gattung Laportea enthaltend

Das Peptidtoxin der Pflanzen der Gattung Dendrocnide und/ oder Pflanzen der Gattung Urera und/ oder Pflanzen der Gattung Nanocnide und/ oder Pflanzen der Gattung Girardinia ist in der Lage, Zellen eines Tumors zu zerstören. Durch die Kombination mit dem Gift der Pflanzen der Gattung Laportea wird das tumorzerstörende Gift an der Blut-Hirn-Schranke vorbei in das Gehirn transportiert und kann dort den Hirntumor bekämpfen. Das Gift der Pflanzen der Gattung Laportea wirkt somit als Durchdringungssubstanz für die Blut-Hirn-Schranke. Erst diese besondere Kombination ermöglicht daher eine Zerstörung von Tumorgewebe im Gehirn. Unerwarteterweise wurden die Peptidtoxine von Laportea durch die Peptidtoxine von Dendrocnide und/ oder Urera und/ oder Nanocnide und/ oder Girardinia nicht abgebaut, so dass die Laportea-Peptidtoxine als Transfersubstanzen für die weiteren Tumorzellen zerstörenden Toxine die Blut-Hirn-Schranke überwinden und, in Verbindung mit den Peptidtoxinen von Dendrocnide und/ oder Urera und/ oder Nanocnide und/ oder Girardinia, die Tumoren im Hirnbereich, beispielsweise Oligodendrozyten, zerstören konnten.

Die Gewinnung der Peptidtoxine kann in an sich bekannterweise erfolgen. Hierzu wird voll inhaltlich auf die Beschreibung der PCT/EP00/12902 verwiesen. Durch verschiedene chromatographische Verfahren, beispielsweise durch HPLC-Techniken, können die jeweiligen Gesamtgiftgemische in einzelne Fraktionen aufgetrennt und die Fraktionen dann auf ihre Wirksamkeit hin untersucht werden. Die tumorzellzerstörende Wirkung einzelner Peptidtoxinfraktionen kann in Zellkulturversuchen bestimmt werden. Die Wirksamkeit der tumorzellzerstörenden Peptidtoxinfraktionen in Verbindung mit der Blut-Hirn-Schranken-Durchdringungssubstanz aus Laportea wird dann in an sich bekannten Tiermodellen und Zellversuchen bestimmt. Beispiele hierfür sind in der PCT/EP00/12902 angegeben, auf die hier voll inhaltlich Bezug genommen wird und deren Inhalt in vollem Umfang in die vorliegende Anmeldung mit aufgenommen wird. Wie in der PCT/EP00/12902 anhand von Sicarius-Peptidtoxinen beschrieben, kann der Fachmann auch für die Peptidtoxine aus den Pflanzengattungen Dendrocnide, Urera, Nanocnide und Girardinia und aus dem Gesamtgiftcocktail7-extrakt von Pflanzen der Gattung Laportea die wirksamen Bestandteile isolieren und wie oben dargestellt testen. Hierzu wird auch auf die PCT/EP2006/063281 verwiesen, deren Inhalt ebenfalls in vollem Umfang in die vorliegende Anmeldung mitaufgenommen wird und auf die die Anmelderin sich hier bezieht. Diese Anmeldung zeigt die Verwendung von Peptidtoxinen aus Laportea, welche in der Lage sind, die Blut-Hirn-Schranke zu durchdringen und gleichzeitig die Befähigung aufweisen, andere Peptidtoxine, nämlich die aus Dendrocnide, Urera, Nanocnide, Girardinia, durch die Blut-Hirn-Schranke hindurchdringen zu lassen.

Bei Laportea kann, alternativ zu den Peptidtoxinfraktionen, auch das Gesamtgift/der Gesamtgiftextrakt der Pflanzen eingesetzt werden. Bevorzugt werden nur diejenigen Fraktionen aus dem Gesamtgiftcocktail/Gesamtgiftextrakt von Laportea in der pharmazeutischen Zusammensetzung eingesetzt, welche die Wirkstoffe enthalten, die zur Passage der Blut-Hirn-Schranke befähigt sind bzw. es den Antitumor-Wirkstoffen aus Dendrocnide und/ oder Urera und/ oder Nanocnide und/ oder Girardinia ermöglichen, diese Barriere zu überwinden.

Gemäß einer bevorzugten Weiterbildung der vorliegenden Erfindung enthält die pharmazeutische Zusammensetzung zusätzlich inaktivierten Parapoxvirus ovis, bevorzugt des Stammes D1701 Hierbei handelt es sich um inaktivierten Parapoxvirus ovis des Stammes D1701, dem zusätzlich Polygeline und Aqua ad injectionem zugesetzt wurde.

Gemäß einer weiteren bevorzugten Weiterbildung der vorliegenden Erfindung werden die Pflanzen der Gattung Dendrocnide ausgewählt aus der Gruppe, bestehend aus den Arten Dendrocnide corallodesme und/ oder Dendrocnide cordata und/ oder Dendrocnide cordifolia und/ oder Dendrocnide excelsa und/ oder Dendrocnide gigantea und/ oder Dendrocnide meyeniana und/ oder Dendrocnide moroides und/ oder Dendrocnide peltata und/ oder Dendrocnide sinuata und die Pflanzen der Gattung Urera ausgewählt aus der Gruppe, bestehend aus den Arten Urera caracasana und/ oder Urera baccifera und/ oder Urera expansa und/ oder Urera kaalae Urera nitida und/ oder Urera simplex und die Pflanzen der Gattung Nanocnide ausgewählt aus der Gruppe, bestehend aus den Arten Nanocnide japonica und/ oder Nanocnide lobata und/ oder Nanocnide closii und die Pflanzen der Gattung Girardinia ausgewählt aus der Gruppe, bestehend aus den Arten Girardinia bullosa und/ oder Girardinia diversifolia.

Die Pflanzen der Gattungen Dendrocnide und/ oder Urera und/ oder Nanocnide und/ oder Girardinia und/ oder Laportea werden bevorzugt wie folgt extrahiert, um den Gesamtgiftcocktail der Pflanzen zu erhalten:
Die Blätter der Pflanzen werden bei Temperaturen von 24 bis 31 Grad Celsius geerntet. Die Blätter werden mit gängigen Peptidlösungsmitteln extrahiert. Der hierbei gewonnene Gesamtgiftcocktail wird über ein säulenchromatografisches und/ oder gängige andere, an sich bekannte Verfahren in seine einzelnen Bestandteile zerlegt.

Im Beispiel 2 wird die Gewinnung von Dendrocnide-Peptidtoxin, Urera-Peptidtoxin, Nanocnide-Peptidtoxin und Girardinia-Peptidtoxin näher beschrieben. Die Einzelheiten zur Gewinnung von Peptidtoxinen aus Dendrocnide, Urera, Nanocnide, Girardinia, die ebenfalls auf die Gewinnung von Peptidtoxinen aus Laportea anwendbar sind, sind in der PCT/EP00/12902 angegeben, so dass der Fachmann anhand dieses Standes der Technik problemlos die wirksamen Fraktionen der Pflanzen (Urticaceae)gifte erhalten kann.

Gemäß einer anderen bevorzugten Weiterbildung der vorliegenden Erfindung ist das Peptidtoxin aus Pflanzen der Gattung Dendrocnide das HTDen1 und/ oder das HTDen2 Peptidtoxin. Im Beispiel 2 ist die Herstellung dieser Peptidtoxine näher beschrieben.

Gemäß einer anderen bevorzugten Weiterbildung der vorliegenden Erfindung enthält die Zusammensetzung zusätzlich Lachesis D6. Das Lachesis D6 (Lachesis bedeutet Grubenotter) der DHU (Deutsche Homöopathische Union) wird bevorzugt als Lösungsmittel für das Dendrocnide-Peptidtoxin und/ oder das Urera-Peptidtoxin und/ oder das Nanocnide-Peptidtoxin und/ oder das Girardinia-Peptidtoxin verwendet. Insbesondere bei der Herstellung der Zusammensetzung kann Lachesis D6 vorteilhaft Anwendung finden.

Gemäß einer anderen bevorzugten Weiterbildung der vorliegenden Erfindung sind die Pflanzen der Gattung Laportea ausgewählt sind aus der Gruppe, bestehend aus den Arten Laportea bulbifera, Laportea canadensis, Laportea cuspidata, Laportea grossa, Laportea interrupta, Laportea mooreana, Laportea ruderalis, Laportea septentrionalis.

Gemäß einer besonders bevorzugten Weiterbildung der vorliegenden Erfindung sind die Pflanzen der Gattung Laportea bevorzugt die Arten Laportea bulbifera, Laportea canadensis und Laportea ruderalis.

Auch die Mischungen unterschiedlicher Gifte der Gattungen Dendrocnide oder Urera oder Nanocnide oder Girardinia kann gemäß einer anderen Weiterbildung der vorliegenden Erfindung erfolgen. Die Mischung kann dabei unterschiedliche Arten der Gattung Dendrocnide oder Urera oder Nanocnide oder Girardinia oder Laportea betreffen oder auch Mischungen verschiedener Populationen einer Art.

Ein zweiter Aspekt der vorliegenden Erfindung betrifft die Verwendung einer pharmazeutischen Zusammensetzung, wie sie oben beschrieben ist, zur Herstellung eines Medikaments zur Behandlung von Hirntumoren und Glioblastomen, insbesondere zur Behandlung eines Oligodendrogliom.

Durch die erfindungsgemäße Kombination von Gift oder Teilen des Gifts von Pflanzen der Gattung Dendrocnide oder Urera oder Nanocnide oder Girardinia mit einem Gift oder Teilen des Giftes von Pflanzen der Gattung Laportea wird die Blut-Hirn-Schranke überwunden. Das Gift der Laportea-Pflanzen dient dabei als Durchdringungssubstanz, die ein Durchdringen der Schranke ermöglicht.

Ein anderer Aspekt der vorliegenden Erfindung betrifft ein Verfahren zum Herstellen einer Zusammensetzung mit den Schritten:

Bereitstellen einer isotonischen (0,9%-igen) Natriumchloridlösung mit Dendrocnide-Peptidtoxin und/ oder Urera-Peptidtoxin und/ oder Nanocnide-Peptidtoxin und/ oder Girardinia-Peptidtoxin
- wahlweise Zugabe eines weiteren Dendrocnide-Peptidtoxins und/ oder Urera-Peptidtoxins und/ oder Nanocnide-Peptidtoxins und/ oder Girardinia-Peptidtoxins zu der Natriumchloridlösung; und
- Zugabe eines Gesamtgiftes oder von Peptidtoxinen von Pflanzen der Gattung Laportea mit einem Molekulargewicht von 5 kDa bis 130 kDa und der Fähigkeit zur Durchdringung der Blut-Hirn-Schranke;
- Vermischen der Bestandteile, insbesondere durch Schütteln der Mischung.

Gemäß einer bevorzugten Weiterbildung dieses Aspektes wird zusätzlich vor dem Vermischen ein Immunmodulator zugegeben, bevorzugt Parapoxvirus ovis, insbesondere bevorzugt vom Stamm D1701.

Gemäß einer anderen bevorzugten Weiterbildung dieses Aspektes ist mindestens eines der Dendrocnide-Peptidtoxine und/ oder Urera-Peptidtoxine und/ oder Nanocnide-Peptidtoxine und/ oder Girardinia-Peptidtoxine in Lachesis D6 gelöst, bevor es zur NaCl-Lösung zugegeben wird, bevorzugt beide Sicarius-Peptidtoxine.

Gemäß einer anderen bevorzugten Weiterbildung dieses Aspektes ist das erste Dendrocnide-Peptidtoxin ein Dendrocnide-Peptidtoxin HTDen1 und/ oder das zweite Dendrocnide-Peptidtoxin ein Dendrocnide-Peptidtoxin HTDen2.

Weiterhin bevorzugt werden in der erfindungsgemäßen pharmazeutischen Zubereitung Peptidtoxine aus Dendrocnide-Arten als Durchdringungssubstanzen eingesetzt, welche die Aufnahme der Peptidtoxine in die Tumorzelle erleichtern. Derartige Substanzen und ihre Gewinnung werden beispielsweise in der PCT/EP00/12902 beschrieben.

Ein weiterer Aspekt der vorliegenden Erfindung betrifft ein Kit of parts zur Behandlung von Gehirntumoren und Glioblastomen, umfassend eine pharmazeutische Zusammensetzung wie oben beschrieben und einen Extrakt aus Weihrauch und/ oder Bambus. Insbesondere bevorzugt wird hier der Extrakt aus Weihrauch- und/ oder Bambusblättern verwendet.

Die Hauptbestandteile des Weihrauchs sind Harze, in denen Boswelliasäuren und ätherische Öle enthalten sind. Neben den Boswelliasäuren, die zur Gruppe der pentacyclischen Triterpene gehören, findet man auch ein tetracyclisches Triterpen, die Tirucallsäure, in den Harzen.

Bevorzugt wird in der vorliegenden Erfindung das Präparat H15 Ayurmedica verwendet. Dabei handelt es sich um Tabletten, die jeweils 400 mg standardisierten Trockenextrakt aus Boswellia serata enthalten. Die Dosierung wird je nach Bedarf angepasst. Sie kann sehr breit variieren und liegt üblicherweise im Bereich von 3 Tabletten pro Woche bis hin zu 25 Tabletten pro Tag, bevorzugt 10 Tabletten pro Woche bis 20 Tabletten pro Tag.

Der Weihrauchextrakt wird als Alternative zu Cortison verwendet, um Ödeme im Hirn bei der Behandlung mit der pharmazeutischen Zusammensetzung gemäß der vorliegenden Erfindung zu verhindern. Ödeme treten vermehrt bei der Behandlung von Hirntumoren auf, insbesondere wenn diese schnell zerstört werden, d.h., wenn sie schnell abnehmen.

Im Folgenden werden die unterschiedlichen Giftstoffe detailliert erläutert.

Die Tumorzellen werden durch Peptidtoxine, die aus Gift von Pflanzen der Gattungen Dendrocnide und/ oder Urera und/ oder Nanocnide und/ oder Girardinia, erhalten werden können, zerstört. Bevorzugt werden die Arten Dendrocnide corallodesme, Dendrocnide cordata, Dendrocnide cordifolia, Dendrocnide excelsa, Dendrocnide gigantea, Dendrocnide meyeniana, Dendrocnide moroides, Dendrocnide peltata, Dendrocnide sinuata, Urera caracasana, Urera baccifera, Urera expansa, Urera kaalae, Urera nitida, Urera simplex, Nanocnide japonica, Nanocnide lobata, Nanocnide closii, Girardinia bullosa, Girardinia diversifolia.

Die Peptidtoxine können durch bekannte Trennmethoden, wie beispielsweise Gel-Elektrophorese oder Chromatographie, insbesondere Säulenchromatographie, gewonnen werden. Die Gewinnung des Roh-Giftcocktails/-extraktes der Pflanzen ist unten beschrieben.

Für die Überwindung der Blut-Hirn-Schranke werden bevorzugt Wirkstoffe aus dem Gift von Pflanzen der Gattung Laportea gewonnen. Diese gehören zur Familie der Brennnesselgewächse (Urticaceae). Sie sind in den unterschiedlichsten Gebieten zu finden, zum Teil kommen sie auch in den Städten vor. Viele Arten bevorzugen Wald als Lebensraum.

Es werden bevorzugt folgende Arten verwendet:
1. a) Laportea bulbifera
2. b) Laportea canadensis
3. c) Laportea cuspidata
4. d) Laportea grossa
5. e) Laportea interrupta
6. f) Laportea mooreana
7. g) Laportea ruderalis
8. h) Laportea septentrionalis

Die beiden Arten Laportea bulbifera und/ oder Laportea ruderalis werden insbesondere bevorzugt zur Gewinnung der Peptidsubstanzen zur Überwindung der Blut-Hirn-Schranke eingesetzt, wenn Kinder behandelt werden sollen.

Insbesondere bevorzugt bei der Zusammensetzung gemäß der vorliegenden Erfindung sind die Arten Laportea bulbifera und Laportea grossa.

Die Laportea (Laportea canadensis), wie sie in der Homöopathie verwendet wird, kommt vorwiegend in Amerika entlang der Pazifikküste bis nach Kanada vor.

Laportea bulbifera kommt in Sri Lanka, Indien, Bhutan, Myanmar, Thailand, Vietnam, China, Korea, Russland, Japan und Indonesien vor.

Latrodectus cuspidata kommt in China, Japan, Korea und Myanmar vor.

Laportea grossa wird in R.S.A. gefunden.
Laportea interrupta findet sich in Indien, Sri Lanka, Indonesien, Myanmar, Thailand, Malaysia, Vietnam, Yunnan, Taiwan und Australien
Laportea mooreana
Laportea ruderalis ist von den Salomonen, der Weihnachtsinsel, Mikronesien, Französisch-Polynesien, Samoa, Tonga und den Cookinseln bekannt
Laportea septentrionalis

Das Laportea-Gift besteht aus 5 bis 14 verschiedenen Proteinen mit Molekulargewichten von 15 kDa bis 100 kDa. Es wird hierbei der Giftcocktail oder Teile des Giftcocktails der unter a) bis h) genannten Laportea-Arten verwendet und insbesondere die Arten Laportea bulbifera und Laportea grossa. Die unter a) und g) genannten Laportea-Arten sind auch bei Kindern bis zum Alter von etwa 12 Jahren einsetzbar.

Die erfindungsgemäß für die pharmazeutische Zusammensetzung verwendeten Substanzen können auf natürlichem Weg von den Pflanzen gewonnen werden. Diese Gifte wurden ursprünglich zur Verteidigung gegen Fressfeinde entwickelt. Diese natürliche Wirkweise kann durch eine funktionserhaltende, schonende Gewinnung des Giftgrundstoffes (z.B. durch manuelle Ernte der Blätter) erhalten werden.

Gemäß einer Ausführungsform der Erfindung ist eine manuelle Blattgewinnung von den Pflanzen vorgesehen. Dadurch werden echte, unverfälschte native Gifte erhalten. Standardmäßig kann eine Analyse und/ oder Qualitätskontrolle des Rohgiftgemisches über elektrophoretische Verfahren erfolgen.

Die Raumtemperatur beträgt meist etwa 25 bis 31 Grad, die Luftfeuchtigkeit 50 % bis 90 %. Die Tageszeit spielt keine Rolle.

Es ist auch möglich, die beschriebenen Wirkstoffe, die in den Giften der unterschiedlichen Pflanzen enthalten sind, chemisch-synthetisch oder durch gentechnologische Methoden in rekombinierter Form herzustellen. Wie bei chemischen Substanzen üblich, umfasst die vorliegende Erfindung auch Derivate und Salze der erfindungsgemäß bereitgestellten Substanzen. Beispielsweise kann das Peptidtoxin ein oder mehrere, Substitutionen und/ oder Deletionen von Aminosäuren umfassen, wobei natürlich sichergestellt sein muss, dass die erfindungsgemäße medizinische Wirkung erhalten bleibt. Die Gewinnung des beschriebenen Wirkstoffs erfolgt durch in der chemischen Verfahrenstechnik übliche Methoden.

Die Wirkstoffe der vorliegenden Erfindung werden vorzugsweise in Form einer solchen pharmazeutischen Zusammensetzung verwendet, in der sie mit geeigneten Trägem oder Trägerstoffen in Dosen vermischt werden, so daß die Erkrankung behandelt oder zumindest gelindert wird. Eine derartige Zusammensetzung kann (zusätzlich zu den Wirkstoffen und dem Träger) Verdünnungsmittel, Füllmaterialien, Salze, Puffer, Stabilisatoren, Solubilisierungsmittel und andere Materialien enthalten, die in der Technik wohlbekannt sind. Der Begriff "pharmazeutisch verträglich" ist als ein nichttoxisches Material definiert, das die Wirksamkeit der biologischen Aktivität des aktiven Inhaltsstoffes bzw. Wirkstoffes nicht stört. Die Auswahl des Trägers hängt vom Verabreichungsweg ab.

Die pharmazeutische Zusammensetzung kann zusätzlich weitere Mittel enthalten, die die Aktivität des Wirkstoffes steigern oder dessen Aktivität oder Verwendung bei der Behandlung ergänzen. Derartige zusätzliche Faktoren und/ oder Mittel können in der pharmazeutischen Zusammensetzung enthalten sein, um eine synergistische Wirkung zu erzielen oder um Nebenwirkungen bzw. unerwünschte Wirkungen zu minimieren.

Techniken zur Formulierung bzw. Zubereitung und Verabreichung der Verbindungen der vorliegenden Anmeldung sind in "Remington's Pharmaceutical Sciences", Mack Publishing Co., Easton, PA, letzte Ausgabe, zu finden. Eine therapeutisch wirksame Dosis bezieht sich ferner auf eine Menge der Verbindung, die ausreicht, um eine Verbesserung der Symptome zu erreichen, beispielsweise eine Behandlung, Heilung, Prävention oder Verbesserung derartiger Zustände. Geeignete Verabreichungswege können beispielsweise orale, rektale, transmucosale oder intestinale Verabreichung und parenterale Verabreichung einschließen, einschließlich intramuskulärer, subkutaner, intramedulärer Injektionen ebenso wie intrathekaler, direkt intraventrikulärer, intravenöser, intraperitonealer oder intranasaler Injektionen. Die subkutane oder orale Verabreichung an einen Patienten wird bevorzugt.

Verabreicht werden 1-5 ml, bevorzugt 2 - 4 ml der Zusammensetzung im Abstand von etwa 1-4 Tagen. Die Menge an verabreichter Zusammensetzung kann jedoch durch einen Fachmann entsprechend angepasst werden. Die Anpassung kann auch während der Behandlung erfolgen.

Bei den herkömmlichen Behandlungsmethoden bilden sich sowohl beim Primärtumor als auch beim Sekundär- oder Rezidivtumor gerne Ödeme, welche verstärkte Druckschmerzen auslösen. Gegen die Ödembildung wird häufig Cortison, oft auch sehr hoch dosiert, verwendet. Cortisone können jedoch ebenfalls Tumore auslösen. Zudem kann Cortison in seiner Wirkweise stark abhängig machen, so dass beim Absetzen meist ein langsames Ausschleichen in vielen, sich reduzierenden Teildosen notwendig ist.

Die Erfinder der vorliegenden Erfindung haben zudem festgestellt, dass die erfindungsgemäßen Zusammensetzungen bei ihrer Durchgängigkeit ins Gehirn durch Cortisone gestört werden. Überraschenderweise haben die Erfinder jedoch festgestellt, dass als vollwertiger Ersatz gegen die Ödembildung Weihrauch- und/ oder Bambusextrakt verwendet werden kann.

Wie bereits oben erwähnt, kommen bevorzugt H15 Präparate als Weihrauchextrakt in Betracht. Als Bambusextrakt, insbesondere der Extrakt von Bambusblättern in Betracht. Es können jedoch auch direkt getrocknete Weihrauchblätter und/ oder Bambusblätter in Kapselform in Kombination mit der pharmazeutischen Zusammensetzung gemäß der vorliegenden Erfindung verabreicht werden.

Im Folgenden wird anhand von Beispielen die Herstellung und die Wirkweise der pharmazeutischen Zusammensetzung gemäß der vorliegenden Erfindung dargelegt. Diese Beispiele sollen lediglich exemplarisch sein und die Erfindung in keiner Weise beschränken.

Zur Herstellung einer pharmazeutischen Zusammensetzung in Form einer Injektionslösung oder Lösung zur oralen Einnahme wird zunächst in 30 ml einer 0,9%-igen NaCl-Lösung 1 ml einer gesättigten Lösung des Dendrocnide-Peptidtoxins HTDen1 in Lachesis D6 DHU (Deutsche Homöopathische Union) eingebracht. Anschließend gibt man 1 ml einer gesättigten Dendrocnide-Peptidtoxin-HTDen2-Lösung in Lachesis D6 DHU zu. Bevorzugt werden beide Schritte bei Raumtemperatur durchgeführt.

Dieser Mischcocktail wird durch Zugabe einer Substanz von Laportea bulbifera oder Laportea septentrionalis vervollständigt. Dabei können auch die Gifte verschiedener Populationen von Laportea spp. verwendet werden.

Anschließend wird die resultierende Mischung 10-mal zum Erdmittelpunkt hin geschüttelt.

Die entstehende Zusammensetzung kann bei etwa 7°C unter Ausschluss von Licht etwa 12 Monate aufbewahrt werden. Der experimentell bestimmte (elektrophoretisch) Wirkungsverlust beträgt nach 12 Monaten etwa 4 %.

In einer Zellkulturflasche (1250 ml) wird die Wirksamkeit der unterschiedlichen Giftextrakte anhand einer Zellauszählung nach 24, 48 und 100 Stunden bestimmt. Dabei ist ersichtlich, dass die Population der Zellen nur unwesentlich abnimmt. Eine Abnahme der Zellpopulation in vitro entspricht einer Verringerung des Tumors in vivo.

Es zeigt sich, dass nach zunehmender Inkubation der Zellen kaum noch Zellwachstum stattfindet. In der Regel tötet das Dendrocnide-Toxin, das Urera-Toxin, das Nanocnide-Toxin, das Girardinia-Toxin die Tumorzellen zunehmend ab. Als Durchdringungsenzym wird ein Peptidtoxin von Dendrocnide verwendet. Diese Durchdringungssubstanz steht in ausreichender Menge zur Verfügung und erleichtert die Diffusion der Peptidtoxine wie beispielsweise des Dendrocnide-Toxins in das Gewebe und die Zellen.

### Therapiebeispiele

Zur Therapie von Hirntumoren wird den Patienten alle zwei oder drei Tage eine subkutane Injektion oder eine oral einzunehmende Lösung von 2-4 ml einer erfindungsgemäßen Zusammensetzung, je nach Schwere der Erkrankung, verabreicht. Der Therapieverlauf wird mittels Positronenemissionstomographie (PET) und über den Tumormarker LSA (lipidgebundene Sialinsäure) überwacht. Entsprechend kann die Therapie, wenn nötig angepasst werden.

Zusätzlich werden täglich bis zu 20 Tabletten H15 oder 3 Kapseln Weihrauchblätter eingenommen, um eine Ödembildung, welche auch bei zu schneller Tumorzerstörung entstehen kann, zu unterbinden.

In den Kapseln, die Weihrauchblätter enthalten, sind daneben bevorzugt trockene Extrakte von Bambusa ventricosa enthalten.

Behandlungsverlauf anhand verschiedener Patientenbeispiele
- Patientin, geboren 1997, Glioblastom diagnostiziert im 5. Lebensjahr. Inoperabel, nur 3 Bestrahlungen bis Juli 2004 erhalten. Im August 2004 Start mit einer Therapie mit der Zusammensetzung gemäß der vorliegenden Erfindung, zunächst täglich 5 Tage lang 2 ml, dann bis August 2008 jeden 2. Tag 2,5 ml, ab September 2008 alle 3 Tage. Seit 2004 September ging das Mädchen wieder in den Kindergarten, dann in die Schule. Die nun junge Frau steht im Berufsleben und fühlt sich gut. Der LSA "startete" bei 27 und hat sich seit November 2005 auf etwa 21,4 eingependelt. 1-mal pro Woche erhält die Frau 2 Weihrauchkapseln, um starker Ödembildung vorzubeugen. Das PET ist seit 2009 unauffällig. Die letzten vermerkten starken Kopfschmerzen waren im Juli 2012.
- Patient, geboren 1968, Astrozytom Grad III, diagnostiziert im April 2016. Kam im Juni 2016 zu einer Freiburger Ärztin, nach seiner 1. Operation. Erhielt nach der Operation 9 Bestrahlungen und begann mit Temozolomid-Chemotherapie, welche er nur schlecht vertrug. Im Computertomogramm zeigte sich bis Januar 2017 eine starke Ausbreitung des Tumors diffus und vom Zelltyp hin zum Glioblastom. Im Mai 2017 wollte der Patient mit einer Brennnesselgifttherapie beginnen. Er begann zunächst mit einer täglichen Dosis von 5 ml vom 15.-22. Mai 2017. Dann wurde bis zum 15. Oktober 2017 täglich 3 mal 4 mL oral eingenommen (Patient hat Angst vor Spritzen). Ab 17. Oktober bis aktuell alle 2 Tage 2,5 ml. Das Allgemeinbefinden des Patienten ist gut und im PET ist derzeit kein Tumornachweis mehr möglich. Der LSA war nach der Operation etwa 28, Ende Oktober 2017 bei 25 und hat sich seit 2019 bei ungefähr 21 eingependelt. Der Patient nimmt 2-täglich seit Mai 2017 5 Tabletten H15.

### Beispiel 2: Allgemeine Darstellung eines säulenchromatographischen Trennverfahrens

Zur Auftrennung der wirksamen Peptidtoxinfraktionen aus den Gesamtgiftcocktails von Pflanzen der Gattungen Dendrocnide, Urera, Nanocnide und Girardinia und Laportea können säulenchromatographische Trennverfahren bevorzug eingesetzt werden. Nachfolgend wird eine allgemeine Darstellung eines erfindungsgemäß anwendbaren säulenchromatographischen Trennverfahrens gegeben. Selbstverständlich kann ein Fachmann auch andere Verfahren zur Auftrennung von Peptidgemischen einsetzen. Die einzelnen, durch das Trennverfahren enthaltenen Fraktionen werden auf ihre Fähigkeit, Tumorzellen zu zerstören, entweder auf Zellkulturen oder im Tierexperiment überprüft. Die Verwendbarkeit einzelner Fraktionen aus dem Laportea-Gesamtgiftcocktail als Blut-Hirn-Schranke überwindendes Agens erfolgt bevorzugt im Tierversuchsmodell und in modifizierten Zellversuchen.

Allgemeine Darstellung eines säulenchromatographischen Trennverfahrens:
Zur Trennung in die einzelnen Bestandteile/Fraktionen wird das Gesamtgift in 5 mL Proteinlösungsmittel für Säulenchromatografie, welches sich aus 0,25 M Tris/HCl, pH 6,5 bis 7,3, 1,92 M Glycin in destilliertem und deionisiertem Wasser zusammensetzt, aufgenommen. Es wird mit dem Gesamtgiftcocktail eine gesättigte Lösung in einem Teflonvial hergestellt. Zur homogenen Durchmischung des Gesamtgiftcocktails mit dem Proteinlösungsmittel wird 60 Sekunden unter Vermeidung von Schaumbildung auf dem Vortex geschüttelt.

Nach der Homogenisierung wird die Lösung über einen Plexiglastrichter in eine transparente und stehende Plexiglassäule, die einen Innendurchmesser von 1 cm, eine Wandstärke von 2 mm und eine Höhe von 50 cm aufweist und unten konisch bis auf ca. 1,5 mm zuläuft, aber offen ist, eingefüllt. In der Säule ist 15 mL Gel (ACA 34; Matrix 3 % Acrylamid 4 % Agarose; Fraktionierungsbereich (MW): Proteine von 20 - 350 kDa, Ausschlußgrenze: 750 kDa, Kügelchendurchmesser: 60 - 140 µm). Die eingebrachte Giftlösung verdrängt beim Eindringen in das Gel die im Gel befindliche Pufferlösung. Nach vollständigem Eindringen der Giftlösung in das Gel wird portionsweise (ohne das Gel trocken laufen zu lassen) 165 mL Lösungsmittel (0,25 M Tris/HCl, pH 6,5 - 7,3, 1,92 M Glycin) auf die Säule gebracht. Dieses Lösungsmittel verdrängt bei seinem Durchlauf durch das Gel die darin befindliche Giftlösung. Die ersten 15 mL, die unten aus der Säule tropfen sind restlicher Gelpuffer und werden verworfen. Anschließend werden 40 Fraktionen zu 4 mL aufgefangen. Durch die chemischen und physikalischen Eigenschaften dieses Trennsystemes bedingt ergaben sich die 4 mL für die einzelnen Fraktionen. Daß auch wirklich nur eine Komponente pro Fraktion zu finden ist wird mit SDS-PAGE überprüft. Nur eine Bande pro Fraktion.

Für die SDS-PAGE wird als Auftragspuffer für die Fraktion aufs Gel zum Peptidbindungs- und Proteinschutz Roti Load 1 + 2 (Carl Roth GmbH & Co KG, Karlsruhe:SDS-,Glycerol- ,Bromphenolblau, Phosphatpuffer, Roti Load 1 mit Mercaptoethanol, Roti Load 2 ohne Mercaptoethanol) verwendet.

Die einzelnen Fraktionen nach der Säulentrennung werden in sauberen, zuvor sterilisierten und verschraubbaren Teflonvials getrennt aufgefangen.

### Beispiel 3: Herstellung der Urticaceae-Peptidtoxine

Die Blätter der Urticaceae-Arten werden, wie vorstehend näher beschrieben, extrahiert, um den Gesamtgiftcocktail zu gewinnen. Das Gesamtgift wird dann über säulenchromatographische Verfahren, beispielsweise über HPLC, gewonnen. Die Fraktionen 2, 7, 8 und 11 enthalten die hirntumorzerstörenden Substanzen bei den Dendrocnide-Arten Dendrocnide moroides und Dendrocnide excelsa. Die Fraktionen 5 und 7 enthalten die hirntumorzerstörenden Substanzen bei den Urera-Arten Urera baccifera und Urera nitida.

Mit einer SDS-PAG-Elekrophorese ergaben sich mit Coomassie-Blau-Färbung folgende (gemittelte) Molekulargewichte der gefriergetrockneten Substanzen:

### Dendrocnide moroides und Dendrocnide excelsa:

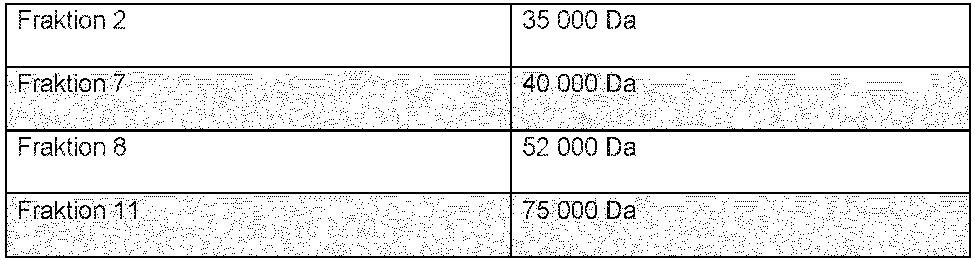

### Urera baccifera und Urera nitida:

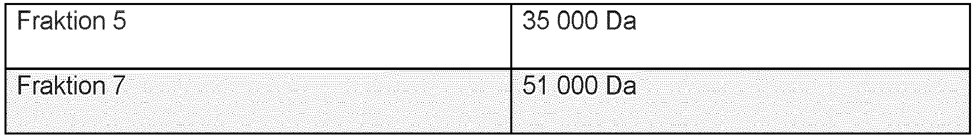

Die Fraktion 4 entspricht dem HT1-Peptidtoxin, die Fraktion 10 dem HT2-Peptidtoxin.

Die Peptidtoxine aus Girardinia und Nanocnide wurden wie HT1 und HT2 auf käuflichen Standard-Glioblastom und Astrozytom-Zellkulturen ausgetestet und zeigten ebenfalls den gewünschten Effekt.

Kit of parts kann erfindungsgemäß als ein Bausatz, eine Vorrichtung und/ oder eine Systemanordnung vorliegen.

## Patentansprüche

1. Pharmazeutische Zusammensetzung zur Behandlung eines Hirntumors, enthaltend in einer pharmazeutisch wirksamen Menge a) Peptidtoxin aus Pflanzen der Gattung Dendrocnide, Pflanzen der Gattung Urera, Pflanzen der Gattung Nanocnide, Pflanzen der Gattung Girardinia; und 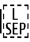 b) Gesamtgift oder Peptidtoxine von Pflanzen der Gattung Laportea mit einem Molekulargewicht von 15 kDa bis 100 kDa und der Fähigkeit zur Überwindung der Blut-Hirn-Schranke.

2. Pharmazeutische Zusammensetzung gemäß Anspruch 1, **dadurch gekennzeichnet, dass** sie zusätzlich inaktivierten Parapoxvirus ovis, vorzugsweise Stamm D1701, enthält.

3. Pharmazeutische Zusammensetzung gemäß einem oder mehreren der vorangegangenen Ansprüche, **dadurch gekennzeichnet, dass** die Pflanzen der Gattung Dendrocnide ausgewählt aus der Gruppe, bestehend aus den Arten Dendrocnide corallodesme und/ oder Dendrocnide cordata und/ oder Dendrocnide cordifolia und/ oder Dendrocnide excelsa und/ oder Dendrocnide gigantea und/ oder Dendrocnide meyeniana und/ oder Dendrocnide moroides und/ oder Dendrocnide peltata und/ oder Dendrocnide sinuata und die Pflanzen der Gattung Urera ausgewählt aus der Gruppe, bestehend aus den Arten Urera caracasana und/ oder Urera baccifera und/ oder Urera expansa und/ oder Urera kaalae und/ oder Urera nitida und/ oder Urera simplex und die Pflanzen der Gattung Nanocnide ausgewählt aus der Gruppe, bestehend aus den Arten Nanocnide japonica und/ oder Nanocnide lobata und/ oder Nanocnide closii und die Pflanzen der Gattung Girardinia ausgewählt aus der Gruppe, bestehend aus den Arten Girardinia bullosa und/ oder Girardinia diversifolia, wobei die Arten Dendrocnide moroides, Dendrocnide excelsa, Urera baccifera, Urera kaalae, Urera nitida und Girardinia diversifolia insbesondere bevorzugt sind.

4. Pharmazeutische Zusammensetzung gemäß einem oder mehreren der vorangegangenen Ansprüche, **dadurch gekennzeichnet, dass** das Peptidtoxid aus Pflanzen der Gattung Dendrocnide das HTDen1- und/ oder das HTDen2-Peptidtoxin ist.

5. Pharmazeutische Zusammensetzung gemäß einem oder mehreren der vorangegangenen Ansprüche, **dadurch gekennzeichnet, dass** sie zusätzlich Lachesis D6 enthält.

6. Pharmazeutische Zusammensetzung gemäß einem oder mehreren der vorangegangenen Ansprüche, **dadurch gekennzeichnet, dass** die Pflanzen der Gattung Laportea ausgewählt sind aus der Gruppe, bestehend aus den Arten Laportea bulbifera, Laportea canadensis, Laportea cuspidata, Laportea grossa, Laportea interrupta, Laportea mooreana, Laportea ruderalis, Laportea septentrionalis

7. Pharmazeutische Zusammensetzung gemäß einem oder mehreren der vorangegangenen Ansprüche, **dadurch gekennzeichnet, dass** die Pflanzen der Gattung Laportea bevorzugt die Arten Laportea bulbifera und/ oder Laportea canadensis und/ oder Laportea ruderalis sind.

8. Pharmazeutische Zusammensetzung gemäß einem oder mehreren der vorangegangenen Ansprüche, **dadurch gekennzeichnet, dass** sie weiterhin Peptidtoxine aus Dendrocnide, Urera, Nanocnide und/ oder Girardinia enthält.

9. Zusammensetzung gemäß einem der Ansprüche 1-8 zur Verwendung in einem Verfahren zur Behandlung von Hirntumoren und Glioblastomen.

10. Verfahren zum Herstellen einer Zusammensetzung gemäß einem der Ansprüche 1-8 mit den Schritten: - Bereitstellen einer isotonischen Natriumchloridlösung mit Dendrocnide-Peptidtoxin und/ oder Urera-Peptidtoxin und/ oder Nanocnide-Peptidtoxin und/ oder Girardinia-Peptidtoxin; wahlweise Zugabe eines weiteren Dendrocnide-Peptidtoxins und/ oder Urera-Peptidtoxins, und/ oder Nanocnide-Peptidtoxins und/ oder Girardinia-Peptidtoxins zu der Natriumchloridlösung; und Zugabe eines Gesamtgiftes/-extraktes oder von Peptidtoxinen von Pflanzen der Gattung Laportea, mit einem Molekulargewicht von 15 kDa bis 100 kDa und der Fähigkeit zur Durchdringung der Blut-Hirn-Schranke; Vermischen der Bestandteile, insbesondere durch Schütteln der Mischung.

11. Verfahren gemäß Anspruch 10, wobei zusätzlich vor dem Schütteln ein Immunmodulator, bevorzugt inaktiviertes Parapoxvirus ovis, zugegeben wrird. 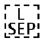

12. Verfahren gemäß Anspruch 10 oder 11, wobei mindestens eines der Dendrocnide-Peptidtoxine und/ oder der Urera-Peptidtoxine und/ oder der Nanocnide-Peptidtoxine und/ oder der Girardinia-Peptidtoxine in Lachesis D6 gelöst sind.

13. Verfahren gemäß einem oder mehreren der Ansprüche 10-12, wobei das erste Dendrocnide-Peptidtoxin ein Dendrocnide-Peptidtoxin HTDen1 ist.

14. Verfahren gemäß einem oder mehreren der Ansprüche 10-13, wobei das zweite Dendrocnide-Peptidtoxin ein Dendrocnide-Peptidtoxin HTDen2 ist.

15. Kit of parts zur Behandlung von Hirntumoren und Glioblastomen umfassend i) eine pharmazeutische Zusammensetzung gemäß einem oder mehreren der Ansprüche 1-8; und ii) Extrakt aus Weihrauch und/ oder Bambus
